# EUROPÄISCHE PATENTSCHRIFT

(11) **EP 0 459 230 B1**
(45) Veröffentlichungstag und Bekanntmachung des Hinweises auf die Patenterteilung: **31.08.1994**
(21) Anmeldenummer: 91107894.7
(22) Anmeldetag: 16.05.1991
(51) Int. Cl.: C07D 307/64, C07D 333/18, A23L 1/226

(54) **Methanthiole, Verfahren zu ihrer Herstellung und ihre Verwendung**
Methanthiols, process for their preparation and their use
Méthanthiols, procédé pour leur préparation et leur utilisation

(30) Priorität: 29.05.1990 DE 4017242
(43) Veröffentlichungstag der Anmeldung: 04.12.1991
(73) Patentinhaber: HAARMANN & REIMER GMBH, D-37601 Holzminden (DE)
(72) Erfinder: Emberger, Roland, Dr., W-3450 Holzminden (DE); Güntert, Matthias, Dr., W-3450 Holzminden (DE); Hopp, Rudolf, Dr., W-3450 Holzminden (DE); Köpsel, Manfred, Dr., W-3450 Holzminden (DE); Thielmann, Thomas, Dr., W-3450 Holzminden (DE); Werkhoff, Peter, Dr., W-3470 Höxter 1 (DE)
(74) Vertreter: Petrovicki, Wolfgang, Dr.

(56) Entgegenhaltungen:
- EP-A- 0 043 486
- EP-A- 0 361 168
- DE-A- 2 437 890
- DE-A- 2 605 286

## Beschreibung

Die Erfindung betrifft neue Methanthiole, ein Verfahren zu ihrer Herstellung und ihre Verwendung als Aromastoffe.

Es wurden neue Methanthiole der Formel 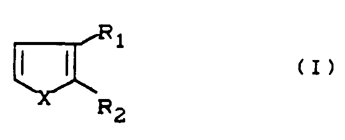
gefunden, in der
- X: für ein Sauerstoff- oder Schwefelatom
und einer der Reste
- R₁ oder R₂: für eine Mercaptomethyl- und der andere für eine Ethylgruppe steht.

Außerdem wurde gefunden, daß diese Methanthiole der Formel (I) wertvolle organoleptische Eigenschaften aufweisen.

Die Erfindung betrifft daher die neuen Methanthiole der Formel (I) und deren Verwendung als Aromastoffe.

Die erfindungsgemäßen Methanthiole werden durch Umsetzung von Hydroxymethyl-Verbindungen der Formel 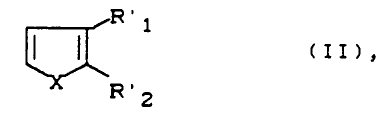
in der
- X: die unter Formel (I) angegebene Bedeutung hat
und einer der Reste
- R'₁ oder R'₂: für eine Hydroxymethyl- und der andere für eine Ethylgruppe steht,
mit Thionylchlorid und anschließender Umsetzung der erhaltenen Chlormethyl-Verbindung mit Thioharnstoff erhalten.

Die Erfindung betrifft daher auch ein Verfahren zur Herstellung von Methanthiolen der Formel (I), das dadurch gekennzeichnet ist, daß man Verbindungen der Formel (II) mit Thionylchlorid in einem inerten Lösungsmittel, vorzugsweise einem Ether, bei Temperaturen von 0°C bis 40°C, vorzugsweise bei Raumtemperatur, umsetzt und die erhaltene Chlormethyl-Verbindung in einem Alkohol, vorzugsweise Methanol oder Ethanol, bei Temperaturen von 20°C bis zur Siedetemperatur des verwendeten Alkohols, vorzugsweise bei der Siedetemperatur, mit Thioharnstoff umsetzt.

Die Ausgangsverbindungen der Formel (II) sind nach üblichen Herstellungsverfahren aus literaturbekannten Verbindungen erhältlich.

Die erfindungsgemäßen Verbindungen sind wertvolle Aromastoffe, die sich durch sehr niedrige Geschmacksschwellenwerte auszeichnen. So liegt zum Beispiel der Geschmacksschwellenwert für (2-Ethyl-furyl-3)-methanthiol, getestet von 5 speziell geschulten Prüfern, bei 0,1 ppt. Die Geschmacksbeschreibungen für einige typische Vertreter der erfindungsgemäßen Verbindungen, bestimmt in 0,5 %iger wäßriger Kochsalzlösung, lauten für

### (3-Ethyl-thienyl-2)-methanthiol:

- bei 1,25 ppt:: fettig, Brühe, Leber, Fülle
- bei 12,5 ppt:: fettig, Huhn, Fleisch, Leber, Röstnote, Brotrinde

### (2-Ethyl-thienyl-3)-methanthiol:

- bei 1,25 ppt:: Kohlrabi, brenzlig, Braten, Röstnote

### (2-Ethyl-furyl-3)-methanthiol:

- bei 0,15 ppt:: Fülle, fettig, Fleischbrühe, Hühnerfett
- bei 1,5 ppt:: Fülle, fettig, Blutnote, Schweinefleisch, Fleischbrühe

Mit ihrem spezifischen Geschmack in Richtung Fleisch wirken die erfindungsgemäßen Verbindungen der Formel (I) in Fleischaromakompositionen geschmacksverstärkend und -abrundend. Aber auch in anderen Aromakompositionen, z.B. Nußaromen, bewirken die erfindungsgemäßen Verbindungen eine Abrundung des Aromas und eine Erhöhung der Geschmacksfülle.

Die unter Verwendung der erfindungsgemäßen Verbindungen hergestellten Aromakompositionen können im gesamten Nahrungsmittel- und Genußmittelbereich, sowie in Tierfutter eingesetzt werden. Insbesondere sind sie geeignet für Fettmassen, Backwaren, Extrusionsprodukte, Fertiggerichte, Fleisch- und Wurstprodukte, Suppen, Soßen, Gemüsekonserven und alle Arten von industriell gefertigtem Tierfutter.

Die erfindungsgemäßen neuen Methanthiole werden in Mengen von 5 ppt bis 1 %, vorzugsweise 100 ppt bis 100 ppm, bezogen auf das verzehrfertige Nahrungsmittel verwendet.

Bei den in den Beispielen verwendeten Prozentangaben handelt es sich um Gewichtsprozente.

### Beispiele

### Beispiel 1

9 ml einer 20 %igen Lösung von n-Butyllithium in Toluol wurden bei -78°C unter trockenem Stickstoff so langsam tropfenweise mit einer Lösung von 3 g 2-Brom-3-ethylthiophen (Chem. Scr. 1974, 217) in 10 ml Ether versetzt, daß die Temperatur -60°C nicht überschritt. Dann wurde mit 1,2 g Paraformaldehyd versetzt und 1,5 Stunden auf Raumtemperatur erwärmt. Das Reaktionsgemisch wurde auf verdünnte Salzsäure gegeben und mit Ether extrahiert. Nach dem Abdestillieren des Lösungsmittels verblieben 2,2 g Rohprodukt, die gemäß Gaschromatogramm 65,7 % 3-Ethyl-2-hydroxymethyl-thiophen enthielten.

1,4 g dieses Rohproduktes und 1 g Pyridin in 25 ml Ether wurden mit 1,26 g Thionylchlorid versetzt. Nach 4-stündigem Rühren bei Raumtemperatur wurde das Reaktionsgemisch in verdünnte Salzsäure gegossen und mehrfach mit Ether extrahiert. Die organische Phase wurde neutralgewaschen, getrocknet und eingeengt. Der Rückstand wurde in 50 ml Ethanol gelöst und mit 0,8 g Thioharnstoff 30 Minuten auf Rückflußtemperatur erhitzt. Nach Abziehen des Lösungsmittels wurde der Rückstand mit 50 ml 5-N Natronlauge 1 Stunde bei 60°C unter Stickstoff gerührt. Dann wurde mit verdünnter Salzsäure angesäuert und mit Ether extrahiert. Das nach Abziehen des Lösungsmittels verbleibende Rohprodukt wurde bei 71°C und 0,3 mbar destilliert. Es wurden 0,2 g (3-Ethyl-thienyl-2)-methanthiol erhalten.

Wurde anstelle von 3-Ethyl-2-hydroxymethyl-thiophen das isomere 2-Ethyl-3-hydroxymethyl-thiophen (US-PS 4 560 701) eingesetzt, so wurde in gleicher Ausbeute (2-Ethyl-thienyl-3)-methanthiol erhalten, Aus 2-Ethyl-3-hydroxymethyl-furan (Farmaco, Ed. Sci. 1982, 398) wurde analog (2-Ethyl-furyl-3)-methanthiol erhalten. Die IR-, NMR- und Massenspektren der Verbindungen stimmten mit den angegebenen Strukturen überein.

### Beispiel 2

Eine Fleischaromakomposition wurde durch Mischen folgender Bestandteile in den angegebenen Gewichtsteilen hergestellt:

| | |
|---|---|
| 50:50 Mischung von Na-Inosinat und Na-Guanilat | 1 |
| Mononatriumglutamat | 19 |
| Milchsäure, sprühgetrocknet | 30 |
| Pflanzenproteinhydrolysat (Type RFB der Food Ingredients Specialities) | 350 |
| Süßmolkepulver | 100 |
| Speisesalz | 500 |
| | 1̅.̅0̅0̅0̅ |

Eine 1 %ige wäßrige Lösung dieser Komposition diente als Kontrollprobe.

Wurden zu der Kontrollprobe 15 ppt (3-Ethyl-thienyl-2)-methanthiol zugegeben, so wurde der Geschmack von einer Testgruppe im Vergleich zur Kontrollprobe als deutlich kräftiger und voller in Richtung Rindfleischbrühe, Fleischsoße und Bratenfleisch mit einer Lebernote im Hintergrund beschrieben.

Bei der Zugabe von 75 ppt (2-Ethyl-furyl-3)-methanthiol zu der Kontrollprobe wird der Geschmack als deutlich voller in Richtung Bratenfleisch beurteilt.

## Patentansprüche

1. Methanthiole der Formel 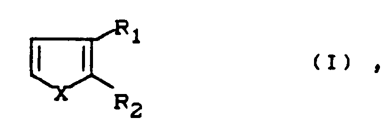 in der
X für ein Sauerstoff- oder Schwefelatom
und einer der Reste
R₁ oder R₂ für eine Mercaptomethyl- und der andere für eine Ethylgruppe steht.

2. Verfahren zur Herstellung der Methanthiole der Formel 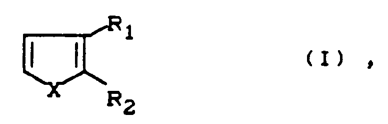 in der
X für ein Sauerstoff- oder Schwefelatom
und einer der Reste
R₁ oder R₂ für eine Mercaptomethyl- und der andere für eine Ethylgruppe steht,
dadurch gekennzeichnet, daß man Verbindungen der Formel 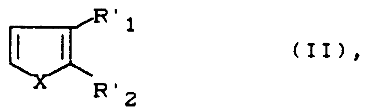 in der
X die unter Formel (I) angegebene Bedeutung hat
und einer der Reste
R'₁ oder R'₂ für eine Hydroxymethyl- und der andere für eine Ethylgruppe steht,
mit Thionylchlorid und die erhaltene Chlormethyl-Verbindung mit Thioharnstoff umsetzt.

3. Verwendung der Methanthiole nach Anspruch 1 als Aromastoffe.

## Claims

1. Methanethiols of the formula 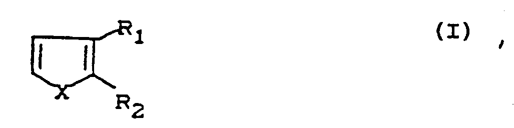 in which
X represents an oxygen or sulphur atom,
and one of the radicals
R₁ or R₂ represents a mercaptomethyl group and the other represents an ethyl group,

2. Process for the preparation of the methanethiols of the formula 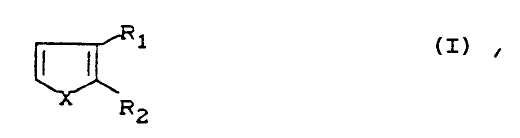 in which
X represents an oxygen or sulphur atom,
and one of the radicals
R₁ or R₂ represents a mercaptomethyl group and the other represents an ethyl group,
characterized in that compounds of the formula 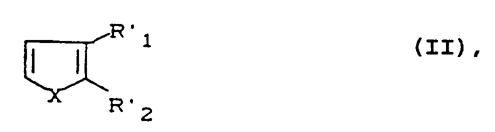 in which
X has the meaning indicated under formula (I)
and one of the radicals
R'₁ or R'₂ represents a hydroxymethyl group and the other represents an ethyl group,
are reacted with thionyl chloride and the resulting chloromethyl compound is reacted with thiourea.

3. Use of the methanethiols according to Claim 1 as flavourings.

## Revendications

1. Méthane-thiols de formule : 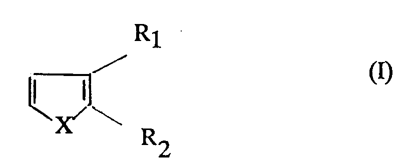 dans laquelle
X représente un atome d'oxygène ou de soufre,
et l'un des symboles R₁ et R₂ représente un groupe mercaptométhyle et l'autre un groupe éthyle.

2. Procédé de préparation des méthane-thiols de formule : 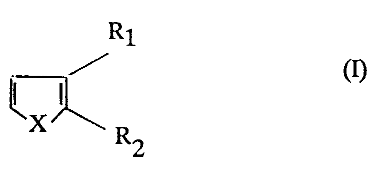 dans laquelle
X représente un atome d'oxygène ou de soufre,
et l'un des symboles R₁ et R₂ représente un groupe mercaptométhyle et l'autre un groupe éthyle,
caractérisé en ce que l'on fait réagir des composés de formule : 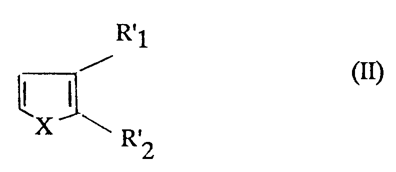 dans laquelle
X a les significations indiquées en référence à la formule I, et
l'un des symboles R'₁ et R'₂ représente un groupe hydroxyméthyle et l'autre un groupe éthyle,
avec le chlorure de thionyle, ce qui donne un dérivé chlorométhylé qu'on fait réagir avec la thiourée.

3. Utilisation des méthane-thiols selon la revendication 1 en tant que matières aromatiques.
